(19) Europäisches Patentamt
European Patent Office
Office européen des brevets

(11) Veröffentlichungsnummer: **0 039 419**
**A1**

(12) # EUROPÄISCHE PATENTANMELDUNG

(21) Anmeldenummer: **81102664.0**

(22) Anmeldetag: **08.04.81**

(51) Int. Cl.³: **C 07 C 119/12**
// A61K7/13, C07C91/44

(30) Priorität: **30.04.80 CH 3331/80**

(43) Veröffentlichungstag der Anmeldung: **11.11.81**
**Patentblatt 81/45**

(84) Benannte Vertragsstaaten: **AT BE CH DE FR GB IT LI LU NL SE**

(71) Anmelder: **LONZA-Werke G.m.b.H., D-7858 Weil am Rhein, Baden (DE)**

(72) Erfinder: **Sappelt, Reinhard, Dr. Dipl.-Chem., Dr. Zimmermann-Strasse 3a, Meersburg (DE)**

(74) Vertreter: **Weinhold, Peter, Dr. Patentanwälte Dr. V. Schmied-Kowarzik et al, Dipl.-Ing. G. Dannenberg Dr. P. Weinhold, Dr. D. Gudel Dipl.-Ing. S. Schubert Siegfriedstr. 8, D-8000 München 40 (DE)**

(54) **Verfahren zur Herstellung von 2,6-Dichlor-5-hydroxy-anilen.**

(57) Verfahren zur Herstellung von 2,6-Dichlor-5-hydroxy-anilen der allgemeinen Formel

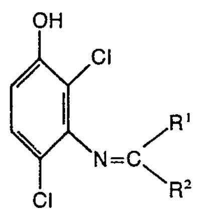

wobei $R^1$ und $R^2$ gleich oder verschieden sein können und Alkylreste mit 1 bis 4 C-Atomen bedeuten, durch Umsetzung von 2,4-Dichlor-3-aminophenol mit Ketonen.

EP 0 039 419 A1

0039419

Verfahren zur Herstellung von 2,6-Dichlor-5-hydroxy-anilen

Die vorliegende Erfindung betrifft ein Verfahren zur Herstellung von 2,6-Dichlor-5-hydroxy-anilen der allgemeinen Formel

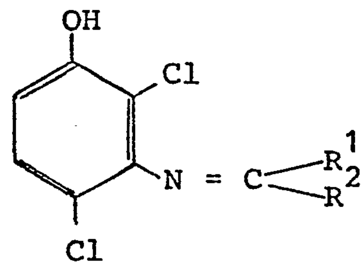

wobei $R^1$ und $R^2$ gleich oder verschieden sein können und Alkylreste mit 1 bis 4 C-Atomen bedeuten.

Diese Verbindungen sind Kupplungskomponenten zur Herstellung von Farbstoffen.

Sie werden erfindungsgemäss durch Umsetzung eines Gemisches aus chlorierten Aminophenolen, welches 2,4-Dichlor-3-aminophenole enthält, mit Verbindungen der allgemeinen Formel

$$R^1\ CO\ R^2,$$

wobei $R^1$ und $R^2$ gleich oder verschieden sein können und Alkylreste mit 1 bis 4 C-Atomen bedeuten, hergestellt.

Verbindungen der Formel $R^1$ CO $R^2$ sind beispielsweise Ketone, wie Azeton, Methyläthylketon, Diisobutylketon.

Die Verbindungen der Formel $R^1$ CO $R^2$ werden vorzugsweise im Ueberschuss angewendet, wobei diese gleichzeitig als Lösungsmittel dienen. Das Verfahren der Erfindung kann auch in Gegenwart anderer Lösungsmittel, wie Alkohole oder chlorierte

Kohlenwasserstoffe, durchgeführt werden.

Die Umsetzung wird zweckmässig in Gegenwart wasserabspaltungsbegünstigender Katalysatoren durchgeführt. Solche sind vorteilhafterweise Mineralsäuren, wie HCl, $H_2SO_4$, sowie Toluolsulfonsäure u.a. Vorzugsweise wird HCl angewendet.

Die anzuwendenden Temperaturen liegen zwischen 0 bis 50°C, vorzugsweise bei 6 bis 12°C.

Der vorteilhafteste Weg, um das 2,6-Dichlor-5-hydroxy-anilen herzustellen, ist der, dass man vom durch Chlorierung von 3-Nitrophenol und nachfolgender Reduzierung der Nitrogruppe erhaltenen Chloramingemisch ausgeht, welches zu je einen Drittel

2,4-Dichlor-3-aminophenol,
4,6-Dichlor-3-aminophenol und
2,4,6-Trichlor-3-aminophenol

enthält.

Ueberraschenderweise hat sich ergeben, dass nur das 2,4-Dichlor-3-aminophenol umgesetzt wird und die gewünschte entsprechende Schiff'sche Base bildet.

Das Verfahren der Erfindung kann als Trennungsverfahren zur Abtrennung von 2,4-Dichlor-3-aminophenol aus dem Chloramingemisch angesehen werden.

Die Chlorierung von 3-Nitrophenol wird zweckmässig mit elementarem Chlor in der Schmelze im Temperaturbereich von 60 bis 100°C, vorzugsweise bei 70 bis 90°C, oder in halogenierten Lösungsmitteln, wie Chloroform, Tetrachlorkohlenstoff, Tetrachloräthan, 1,1,2-Trichlorfluoräthan und o-Dichlorbenzol, bei der Siedetemperatur des Lösungsmittels, vorzugsweise bei 50°C, durchgeführt.

Die Chlorierung kann auch in Wasser in Gegenwart eines Emulgators oder in Gegenwart von Essigsäure bei Temperaturen von 40 bis 90°C, vorzugsweise bei 50 bis 80°C, durchgeführt werden.

Beispiel 1

4200 g Chloramingemisch werden in 10 l Azeton gelöst und unter Kühlung auf 8 bis 10°C gehalten. Während einer Zeit von 6 1/2 Stunden wird in diese Lösung 1000 g HCl-Gas eingeleitet. Anschliessend lässt man noch weitere 3 Stunden nachreagieren und saugt erst dann die ausgefallenen Kristalle ab. Der stark rötlich-braun gefärbte Filterkuchen wird in Azeton aufgeschlämmt, abgesaugt und dreimal mit je 500 ml Azeton, also insgesamt mit 1,5 l Azeton, nachgewaschen. Es wird sofort abgesaugt und abgepresst, so dass der Filterkuchen weitgehend vom Azeton befreit wird.

Die Ausbeute beträgt nach Trocknung im Vakuum 1000 g 2,6-Dichlor-5-hydroxy-N(isopropyliden)anilin mit einem Smp von 177 bis 179°C.

Die Identifizierung erfolgte mittels NMR-Spektroskopie.

Beispiel 2

4200 g Chloramingemisch werden in 10 l Methyläthylketon gelöst und unter Kühlung auf 8 bis 10°C gehalten. Während einer Zeit von 6 1/2 Stunden werden in diese Lösung 1000 g HCl-Gas eingeleitet. Anschliessend lässt man noch weitere 3 Stunden nachreagieren und saugt erst dann die ausgefallenen Kristalle ab.

0039419

Der stark rötlich-braun gefärbte Filterkuchen wird in Methyläthylketon aufgeschlämmt, abgesaugt und dreimal mit je 500 ml Methyläthylketon, also insgesamt mit 1,5 l Methyläthylketon, nachgewaschen. Es wird sofort abgesaugt und abgepresst, so dass der Filterkuchen weitgehend vom Methyläthylketon befreit wird. Die Ausbeute beträgt nach Trocknung im Vakuum 1080 g 2,6-Dichlor-5-hydroxy-N(butyliden-2)anilin mit einem Smp von 171 bis 173°C.

Die Identifizierung erfolgte mittels NMR-Spektroskopie.

---

Patentansprüche

1. Verfahren zur Herstellung von 2,6-Dichlor-5-hydroxyanilen der allgemeinen Formel

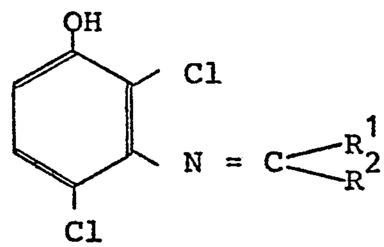

wobei $R^1$ und $R^2$ gleich oder verschieden sein können und Alkylreste mit 1 bis 4 C-Atomen bedeuten, dadurch gekennzeichnet, dass man ein Gemisch aus chlorierten Aminophenolen, welches 2,4-Dichlor-3-Aminophenol enthält, mit Ketonen der allgemeinen Formel

$$R^1 \; CO \; R^2$$

wobei $R^1$ und $R^2$ der oben definierten Bedeutung entsprechen, umsetzt.

2. Verfahren zur Herstellung von 2,6-Dichlor-5-hydroxyanilen gemäss Patentanspruch 1, dadurch gekennzeichnet, dass man die Umsetzung bei Temperaturen von 0 bis 50°C durchführt.

3. Verfahren zur Herstellung von 2,6-Dichlor-5-hydroxyanilen gemäss Patentansprüchen 1 bis 2, dadurch gekennzeichnet, dass man als Verbindung der allgemeinen Formel $R^1$ CO $R^2$ Azeton verwendet.

4. Verfahren zur Herstellung von 2,6-Dichlor-5-hydroxy-anilen gemäss Patentansprüchen 1 bis 3, dadurch gekennzeichnet, dass man als Verbindung der allgemeinen Formel $R^1$ CO $R^2$ Methyläthylketon verwendet.

5. Verfahren zur Herstellung von 2,6-Dichlor-5-hydroxy-anilen gemäss Patentansprüchen 1 bis 4, dadurch gekennzeichnet, dass man die Ketone im Ueberschuss zur Anwendung bringt.

Europäisches Patentamt

# EUROPÄISCHER RECHERCHENBERICHT

0039419 Nummer der Anmeldung

EP 81 10 2664.0

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A1 - 2 509 096 (HENKEL)<br>* Anspruch 7 *<br>-- | 1 |
| P,A | DE - A1 - 2 924 089 (HENKEL)<br>-- | |
| A | DE - C - 693 988 (I.G. FARBENINDUSTRIE)<br>---- | |

**KLASSIFIKATION DER ANMELDUNG (Int. Cl.³)**

C 07 C 119/12
//A 61 K 7/13
C 07 C 91/44

**RECHERCHIERTE SACHGEBIETE (Int. Cl.³)**

C 07 C 91/44
C 07 C 119/06
C 07 C 119/12

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 12-08-1981 | PHILLIPS |